# EUROPÄISCHE PATENTANMELDUNG

(11) **EP 4 714 481 A1**
(43) Veröffentlichungstag der Anmeldung: **25.03.2026**
(21) Anmeldenummer: 24201886.9
(22) Anmeldetag: 23.09.2024
(51) Int. Cl.: A61M 5/36, A61M 5/142

(54) **INFUSIONSPUMPE MIT LUFTBLASENERKENNUNG UND VERFAHREN ZUM BETRIEB EINER INFUSIONSPUMPE ZUR LUFTBLASENERKENNUNG**

(71) Anmelder: B. Braun Melsungen AG, 34212 Melsungen (DE)
(72) Erfinder: SCHWARZ, Jan, 34212 Melsungen (DE); MOELLER, Sebastian, 37235 Hessisch Lichtenau (DE)
(74) Vertreter: Winter, Brandl - Partnerschaft mbB

(57) **Zusammenfassung**

Offenbart sind eine Infusionspumpe (1) mit einer Sensoranordnung (11, 13) und ein Verfahren zum Betrieb der Infusionspumpe (1). Die Infusionspumpe (1) weist eine Infusionsleitung (4) oder eine Aufnahmevorrichtung (10) mit einem Aufnahmebereich (10a) für eine Infusionsleitung (4) auf. Am Außenumfang des Aufnahmebereiches (10a) der Aufnahmevorrichtung (10) oder am Außenumfang der Infusionsleitung (4) sind ein Ultraschallsensor (13) und eine Bildgebungsvorrichtung (11) mit einer Kamera angeordnet. Zumindest die Bildgebungsvorrichtung ist zur Erkennung (20) von Luftblasen oder anderen Gasblasen und vorzugsweise zur Erkennung von Partikeln in einer Infusionsflüssigkeit eingerichtet und ausgelegt ist. Weiterhin ist vorzugsweise zumindest die Bildgebungsvorrichtung (11) zur Erkennung einer Größe und zur Erkennung einer Veränderung der Größe der Gasblasen und/oder zur Erkennung der Bewegung der Gasblasen und der Partikel eingerichtet und ausgelegt. Der vorgesehene Ultraschallsensor (13) übernimmt vorzugsweise eine Initialisierung (18) durch Erkennung der Infusionsflüssigkeit in der Infusionsleitung (4) und gegebenenfalls die Erkennung (20) der Luftblasen oder anderen Gasblasen.

## Beschreibung

### Technisches Gebiet

Die vorliegende Offenbarung betrifft die Luftblasenerkennung und vorzugsweise eine zusätzliche Partikelerkennung einer medizinischen Infusionsflüssigkeit in einer Infusionsleitung, vorzugsweise in einem Infusionsschlauch einer Infusionspumpe, vorzugsweise einer Peristaltikpumpe.

### Hintergrund der Offenbarung

In der Medizin werden flüssige Medikamente mittels Infusionspumpen einem Patienten zugeführt und in dessen Blutkreislauf konstant oder schubweise injiziert. Dabei ist es wichtig, dass keine Luftblasen (oder andere Gasblasen) zusammen mit dem flüssigen Medikament eingebracht werden.

Die betroffene Infusionspumpe kann eine Peristaltikpumpe sein oder aufweisen. In eine derartige Infusionspumpe wird ein Schlauchsegment (Pumpensegment) eingelegt, in dem z.B. mittels äußerer Rollen die Infusionsflüssigkeit weitergefördert wird, und in die ein weiteres Schlauchsegment (Sensorsegment) eingelegt wird, an dem die Luftblasenerkennung durchgeführt wird.

### Stand der Technik

In der EP 3 296 717 A1 der Anmelderin ist ein optischer bildgebender Sensor für einen Schlauch einer Dialysemaschine offenbart, wobei der Sensor Fremdstoffe wie Verunreinigungen und Luftblasen erkennt.

Aus dem Stand der Technik der Anmelderin sind ferner Luftblasensensoren bekannt, die basierend auf der Aussendung eines Ultraschallsignals mit einer Frequenz Luftblasen erkennen können. Dieser Ansatz ist in Abhängigkeit der gewählten Frequenz in seiner Auflösung beschränkt und erkennt daher erst Luftblasen ab einer bestimmten Größe.

Aus der CN 112107758 B ist eine Sensoranordnung für eine Infusionspumpe bekannt, die als Ultraschallsensor für verschiedene Frequenzen ausgelegt ist. Die Luftblasenerkennung mit mehr als einer Frequenz hat den Vorteil, dass unterschiedliche Genauigkeiten möglich sind, dass also unterschiedliche Luftblasengrößen basierend auf der gewählten Frequenz erkannt werden können.

Die Ultraschallsensoren für Infusionspumpen des Standes der Technik sind in der Lage, Luftblasen in einem mit Infusionsflüssigkeit gefüllten Infusionsschlauch zu erkennen, sie sind jedoch aufgrund der gewählten Frequenz limitiert im Hinblick auf die zu erkennende Luftblasengröße.

### Kurzbeschreibung der Offenbarung

Aufgabe der vorliegenden Offenbarung ist es, eine Infusionspumpe und ein Verfahren und ein computerimplementiertes Speichermedium zu schaffen, bei denen die Luftblasenerkennung verbessert ist. Insbesondere soll die Limitierung auf zu erkennende Luftblasengrößen überwunden werden.

Diese Aufgabe wird in Hinblick auf die Infusionspumpe mit der Merkmalskombination des Anspruchs 1 gelöst und im Hinblick auf das Verfahren mit der Merkmalskombination des Anspruchs 11 gelöst und im Hinblick auf ein computerimplementiertes Speichermedium oder auf eine Steuereinheit mit der Merkmalskombination des Anspruchs 16 gelöst.

Die Infusionspumpe gemäß der Offenbarung hat eine Infusionsleitung oder eine Aufnahmevorrichtung mit einem Aufnahmebereich für eine Infusionsleitung. Die Infusionsleitung ist vorzugsweise ein Infusionsschlauch. Am Außenumfang des Aufnahmebereiches und/oder der Infusionsleitung sind eine Bildgebungsvorrichtung mit einer Kamera und weiterhin ein Ultraschallsensor angeordnet, wobei zumindest die Bildgebungsvorrichtung zur Erkennung von Luftblasen oder anderen Gasblasen und vorzugsweise (ergänzend) zur Erkennung von Partikeln einer Infusionsflüssigkeit eingerichtet und ausgelegt ist. Die Infusionsflüssigkeit ist in der Infusionsleitung aufgenommen und kann durch diese strömen aber auch in dieser ruhen. Die Bildgebungsvorrichtung ist in der Lage, durch eine Bildverarbeitung zu erkennen, wann eine Gasblase in der Infusionsflüssigkeit auftritt. Sie kann zudem erkennen, ob sich die Gasblase bewegt, ob sie über die Zeit größer wird oder ob die Luftblase steht. Im letzten Fall kann über die Gasblasenerkennung hinausgehend darauf geschlossen werden, dass sich die Gasblase festgesetzt hat, oder dass die (gesamte) Infusionsflüssigkeit in der Infusionsleitung steht. Im ersten Fall kann ein Alarm verhindert werden. Im zweiten Fall kann diese Zusatzinformation auch von der betroffenen Infusionspumpe als Information zum (übergeordneten) Pumpenbetrieb verwertet werden.

Der ergänzend vorgesehene Ultraschallsensor kann unterschiedlich ausgelegt und eingerichtet sein:
- Der Ultraschallsensor kann ausgelegt und eingerichtet sein nur eine Initialisierung zu übernehmen, wobei er das Vorhandensein der Infusionsflüssigkeit in der Infusionsleitung erkennt. Dazu ist der Ultraschallsensor zur Unterscheidung von Luft und Infusionsflüssigkeit ausgelegt und eingerichtet.
- Der Ultraschallsensor kann ausgelegt und eingerichtet sein nach der Initialisierung bei der Gasblasenerkennung mitzuwirken oder sogar in Sonderfällen (z.B. bei einer lichtabsorbierenden Infusionsleitung und bei einer Bildgebungsvorrichtung, die auf sichtbarem Licht beruht) die Gasblasenerkennung alleine durchzuführen.
- Es ist auch möglich, dass die Bildgebungsvorrichtung selbst zur Initialisierung ausgelegt und eingerichtet ist, und dass der Ultraschallsensor bei der Gasblasenerkennung mitwirkt oder diese in Sonderfällen übernimmt. Dazu sind die Bildgebungsvorrichtung und der Ultraschallsensor zur Erkennung von Gasblasen in der Infusionsflüssigkeit ausgelegt und eingerichtet.

Entsprechend (der oben genannten Fallunterscheidung) kann eine elektronische Steuereinheit der Infusionspumpe wie folgt ausgelegt und eingerichtet sein:
- Die Steuereinheit kann ausgelegt und eingerichtet sein, über den Ultraschallsensor nur eine Initialisierung zu steuern, wobei das Vorhandensein der Infusionsflüssigkeit in der Infusionsleitung erkannt wird, und danach über die Bildgebungsvorrichtung die Gasblasenerkennung zu steuern.
- Die Steuereinheit kann ausgelegt und eingerichtet sein über den Ultraschallsensor die Initialisierung und danach zusammen mit der Bildgebungsvorrichtung die Gasblasenerkennung zu steuern oder sogar in Sonderfällen (z.B. bei einer lichtabsorbierenden Infusionsleitung und bei einer Bildgebungsvorrichtung, die auf sichtbarem Licht beruht) die Gasblasenerkennung nur durch den Ultraschallsensor durchzuführen.
- Die Steuereinheit kann ausgelegt und eingerichtet sein, über die Bildgebungsvorrichtung die Initialisierung zu steuern und danach die Glasblasenerkennung, z.B. redundant, mittels des Ultraschallsensors und der Bildgebungsvorrichtung zu steuern.

Die Aufnahmevorrichtung ist zum übergangsweisen / temporären Aufnehmen und Fixieren der Infusionsleitung, vorzugsweise des Infusionsschlauches eingerichtet und ausgelegt. Dazu hat die Aufnahmevorrichtung den Aufnahmebereich, in den die Infusionsleitung einsetzt und fixiert, z.B. eingespannt und/oder eingeklemmt werden kann. Dann kann die Infusionsleitung als Infusionsschlauch ausgebildet sein und als Einmalartikel beim Wechsel der Infusionsflüssigkeit mit gewechselt werden. Zur Fixierung kann die Aufnahmevorrichtung einen Deckel aufweisen.

Wenn die Kamera auf sichtbarem Licht basiert und insbesondere im Falle des Deckels oder einer Frontklappe eines Gehäuses der betroffenen Infusionspumpe ist es wegen der Abdunkelung bevorzugt, wenn eine Lichtquelle, insbesondere LED, am Außenumfang des Aufnahmebereiches oder der Infusionsleitung angeordnet ist. Vorzugsweise ist die Lichtquelle an einer der Kamera gegenüberliegenden Seite des Aufnahmebereiches oder der Infusionsleitung angeordnet.

Abweichend kann die Kamera auch zur Erfassung von Infrarotstrahlen / Infrarotstrahlung eingerichtet und ausgelegt sein. Dann kann die Bildgebungsvorrichtung auf Grund der Wärmestrahlung unterschiedliche Gasblasen sichtbar machen und erkennen.

Selbstverständlich ist es besonders bevorzugt, wenn die Infusionsleitung lichtdurchlässig ist. Es sind aber auch Einschränkungen bei der Lichtdurchlässigkeit der Infusionsleitung möglich, die mit dem zusätzlichen Ultraschallsensor (zumindest teilweise) kompensiert werden können.

Die Sensoranordnung kann kompakt sein und/oder sie benötigt nur eine gemeinsame Aufnahmevorrichtung für die Infusionsleitung, wenn der Ultraschallsensor benachbart zur Bildgebungsvorrichtung angeordnet ist.

Wenn es bei der Gestaltung der übergeordneten Infusionspumpe von Vorteil ist, kann der Ultraschallsensor auch entlang der Infusionsleitung beabstandet von der Bildgebungsvorrichtung angeordnet sein. Dann hat im Falle des Infusionsschlauches der Ultraschallsensor eine eigene Aufnahmevorrichtung für den Infusionsschlauch.

Der Ultraschallsensor kann für eine Frequenz oder für mehrere bzw. verschiedene Frequenzen ausgelegt sein. Die Luftblasenerkennung lässt sich gemäß einem bevorzugten Ausführungsbeispiel weiter verbessern, wenn der Ultraschallsensor für verschiedene Frequenzen ausgelegt ist.

Eine andere Ausgestaltung berücksichtigt, dass die Bildgebungsvorrichtung anfällig für Verschmutzungen ist, und dass am Markt Infusionsschläuche erhältlich sind, die einen UV-Schutz aufweisen, der aus lichtabsorbierender oder lichtundurchlässiger Beschichtung besteht. Dann kann die Bildgebungsvorrichtung an ihre Grenzen stoßen. Daher ist es besonders bevorzugt, wenn der Ultraschallsensor ebenfalls zur Erkennung von Gasblasen und vorzugsweise zur Erkennung von Partikeln in der Infusionsflüssigkeit eingerichtet und ausgelegt ist. Dann kann der Ultraschallsensor die Erkennung der Gasblasen und vorzugsweise die Erkennung der Partikel in den genannten Fällen übernehmen.

Die Bildgebung ermöglicht zusätzlich durch z.B. Kantenerkennung und/oder Mustererkennung eine Unterscheidung in der im Infusionsschlauch geführten Infusionsflüssigkeit. Durch eine Erkennung einer Veränderung ist eine Erkennung/Schätzung der Größe einer Luftblase oder eines Partikels möglich, nachdem vorzugsweise eine Initialisierung insbesondere durch den Ultraschallsensor erfolgt ist. Eine Initialisierung durch den Ultraschallsensor hat den Vorteil, dass die Bildverarbeitung weniger "leisten" muss, und insbesondere bei UV-beschichteten Infusionsschläuchen kann die Bildgebung dann deutlich besser arbeiten. Da die Bildgebung einen größeren Erkennungsbereich besitzt, kann sie zudem Veränderungen über die Zeit erkennen, um damit die Bewegung von Gasblasen/Partikeln oder das Anwachsen detektieren.

Bei einer bevorzugten Weiterbildung der Infusionspumpe ist die Aufnahmevorrichtung für den Infusionsschlauch an einem Gehäuse der Infusionspumpe befestigt.

Weiterhin kann auch die Frontklappe der Infusionspumpe oder zumindest indirekt auch der Deckel der Sensoranordnung an dem Gehäuse befestigt sein. Insbesondere wenn die Kamera für sichtbares Licht ausgelegt und eingerichtet ist, ist es wegen der Abdunkelung bevorzugt, wenn die oben genannte Lichtquelle vorgesehen ist.

Die betroffene Infusionspumpe ist oder hat bevorzugt eine Peristaltikpumpe (Schlauchpumpe/ Schlauchrollenpumpe/ Schlauchquetschpumpe), die ein Infusionsschlauchsegment (Pumpensegment) hat, in dem mittels äußerer mechanischer Verformung, beispielsweise mittels äußeren Rollen, die Infusionsflüssigkeit (weiter) gefördert wird, insbesondere durch das Infusionsschlauchsegment hindurchgedrückt wird. Das Infusionsschlauchsegment kann mit dem Sensorsegment, an dem die Sensoranordnung angeordnet ist, gemeinsam als einstückiger Infusionsschlauch gebildet sein, oder es handelt sich um zwei oder mehr getrennte Schlauchsegmente, die mittels einer Kupplung verbunden sind.

Die Infusionspumpe hat also vorzugsweise drei Komponenten, die direkt am Infusionsschlauch angeordnet sind, nämlich Mittel zur äußeren mechanischen Verformung, beispielsweise Rollen oder Stößel, der Peristaltikpumpe, die Bildgebungsvorrichtung und den Ultraschallsensor. Diese drei Komponenten können auch beabstandet zueinander an verschiedenen Schlauchsegmenten eines Infusionsschlauches angeordnet sein. Dann hat jede dieser Komponenten eine eigene Aufnahmevorrichtung.

Bei einer Ausgestaltung ist der Ultraschallsensor insbesondere zur Unterscheidung von der Infusionsflüssigkeit und Luft in der Infusionsleitung eingerichtet und ausgelegt. Dann kann die Bildgebungsvorrichtung mit dem Ultraschallsensor initialisiert werden.

Das offenbarungsgemäße Verfahren dient zum Betrieb der vorbeschriebenen Sensoranordnung, und hat die Schritte "Initialisierung" und "Gasblasenerkennung".

Bei einem Ausführungsbeispiel ist vorgesehen, dass die Bildgebungsvorrichtung mit dem Ultraschallsensor initialisiert wird, insbesondere aktiviert/ betriebsbereit gemacht wird.

Nach der Initialisierung können die Bildgebungsvorrichtung und der Ultraschallsensor redundant zueinander Luftblasen oder andere Gasblasen und vorzugsweise Partikel erkennen (detektieren).

Abweichend kann bei der Gasblasenerkennung auch der Ultraschallsensor (lediglich) das Vorhandensein oder die Existenz einer Gasblase, insbesondere Luftblase, detektieren, und die Bildgebungsvorrichtung kann danach das Anwachsen und/oder die Bewegung der Gasblase detektieren.

Falls zwar die Existenz einer Gasblase aber keine Bewegung der Gasblase detektiert wird, kann ein Alarm bzw. eine Fehlermeldung verhindert werden, weil die Gasblase sich am Schlauch festgesetzt hat.

Das offenbarungsgemäße computerimplementierte Speichermedium bzw. die offenbarungsgemäße elektronische Steuereinheit ist dazu ausgelegt und eingerichtet und programmiert, die genannten Verfahrensschritte auszuführen.

### Kurzbeschreibung der Figuren

Figur 1 zeigt eine Infusionspumpe gemäß einem Ausführungsbeispiel der vorliegenden Offenbarung mit weiteren Komponenten in einem schematischen Überblick;
Figur 2 zeigt eine weitere Darstellung des Ausführungsbeispiels der Infusionspumpe aus Figur 1; und
Figur 3 zeigt das Verfahren zum Betrieb der Infusionspumpe aus Figur 1.

### Beschreibung der Ausführungsbeispiele

Nachstehend werden ein Ausführungsbeispiel der Infusionspumpe und drei Ausführungsbeispiele des Verfahrens gemäß der vorliegenden Offenbarung auf der Basis der zugehörigen Figuren beschrieben.

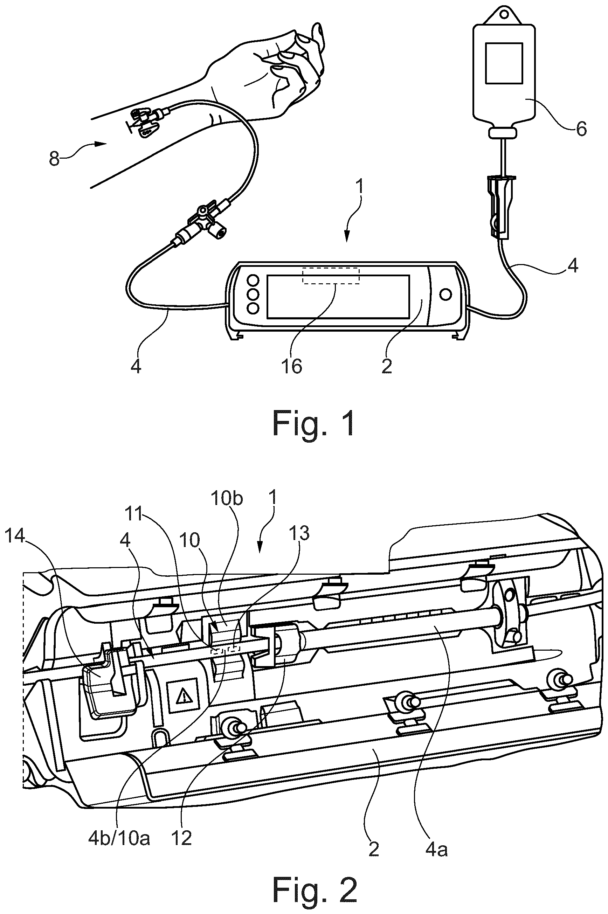

Figur 1 zeigt eine Infusionspumpe 1 gemäß einem Ausführungsbeispiel der vorliegenden Offenbarung. Ihr Gehäuse hat eine Frontklappe 2, die unter anderem einen mehrteiligen Infusionsschlauch 4 teilweise abdeckt. Der Infusionsschlauch 4 hat mindestens zwei getrennte Schlauchsegmente, die mittels jeweiliger Kupplungen verbunden sind. Über den Infusionsschlauch 4 wird ein flüssiges Medikament aus einem Beutel 6 zu einem Zugang eines Patienten 8 gefördert. Dazu ist die Infusionspumpe 1 mit einer Peristaltikpumpe ausgeführt, die mit äußerer mechanischer Verformung an dem Infusionsschlauch 4 angreift und diesen zusammendrückt bzw. quetscht. Die zusammengedrückte Stelle des Infusionsschlauches 4 wandert (in Figur 1 von rechts nach links) entlang dem Infusionsschlauch 4 in Richtung zum Patienten 8.

Figur 2 zeigt eine weitere Darstellung des Ausführungsbeispiels der Infusionspumpe 1 aus Figur 1 mit geöffneter Frontklappe 2. Der Infusionsschlauch 4 erstreckt sich hinter der Klappe 2 durch die Infusionspumpe 1 bzw. durch deren Gehäuse. Dabei weist der Infusionsschlauch 4 das Pumpensegment 4a auf, in dessen Bereich die Peristaltik angreift.

Beim gezeigten Ausführungsbeispiel patientenseitig (also in Figur 2 links) vom Pumpensegment 4a ist ein Sensorsegment 4b des Infusionsschlauchs 4 fixiert, an dessen Außenumfang eine Aufnahmevorrichtung 10 einer Sensoranordnung angeordnet ist. Genauer gesagt hat die Aufnahmevorrichtung 10 zwei Backen 10b, zwischen denen das Sensorsegment 4b des Infusionsschlauchs 4 lösbar fixiert (z.B. eingeclipst) ist.

In die Aufnahmevorrichtung 10 sind einerseits eine Kamera und eine als LED ausgebildete Lichtquelle integriert, die eine Bildgebungsvorrichtung 11 bilden. Weiterhin ist in die Aufnahmevorrichtung 10 ein Ultraschallsensor 13 integriert. Die Bildgebungsvorrichtung 11 und der Ultraschallsensor 13 bilden zusammen die offenbarungsgemäße Sensoranordnung.

Zwischen den beiden Segmenten 4a, 4b des Infusionsschlauchs 4 ist eine Kupplung 12 vorgesehen.

Patientenseitig von der Sensoranordnung mit der Bildgebungsvorrichtung 11 und dem Ultraschallsensor 13 ist am Infusionsschlauch 4 noch eine Klemme 14 (Free Flow Protection Clamp, FFPC) angeordnet. Diese ist zweiteilig, also mit einem inneren am Infusionsschlauch 4 festgelegten Teil und mit einem äußeren am Gehäuse der Infusionspumpe 1 festgelegten Teil.

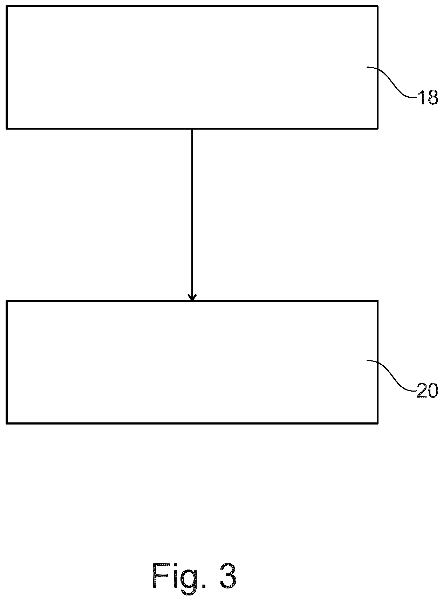

Mit der in den Figuren 1 und 2 gezeigten offenbarungsgemäßen Infusionspumpe 1 können verschiedene Ausführungsbeispiele des offenbarungsgemäßen Verfahrens zur Luftblasen- und Partikelerkennung durchgeführt werden, die von einer (in Figur 1 schematisch gezeigten) elektronischen Steuereinheit 16 entsprechend gesteuert werden. Drei verschiedene Ausführungsbeispiele des Verfahrens sind gemeinsam in Figur 3 schematisch gezeigt. Sie bestehen jeweils aus einer Initialisierung 18 und eine Gasblasenerkennung 20.

Gemäß einem ersten Ausführungsbeispiel steuert die Steuereinheit 16 über den Ultraschallsensor 13 nur die Initialisierung 18, wobei das Vorhandensein der Infusionsflüssigkeit in der Infusionsleitung 4 erkannt wird. Danach steuert die Steuereinheit 16 über die Bildgebungsvorrichtung 11 die Gasblasenerkennung 20.

Gemäß einem anderen Ausführungsbeispiel steuert die Steuereinheit 16 über den Ultraschallsensor 13 die Initialisierung 18. Danach steuert die Steuereinheit 16 über den Ultraschallsensor 13 zusammen mit der Bildgebungsvorrichtung 11 die Gasblasenerkennung 20. Übergangsweise z.B. bei einer lichtabsorbierenden Infusionsleitung 4 kann die Gasblasenerkennung 20 nur durch den Ultraschallsensor 13 erfolgen.

Gemäß einem weiteren Ausführungsbeispiel steuert die Steuereinheit 16 über die Bildgebungsvorrichtung 11 die Initialisierung 18 und danach die Gasblasenerkennung 20 des Ultraschallsensors 13 zusammen mit der Bildgebungsvorrichtung 11.

Offenbart sind eine Infusionspumpe 1 mit einer Sensoranordnung 11, 13 und ein Verfahren zum Betrieb der Sensoranordnung 11, 13. Die Sensoranordnung 11, 13 weist eine Infusionsleitung 4 oder eine Aufnahmevorrichtung 10 mit einem Aufnahmebereich 10a für eine Infusionsleitung 4 auf. Am Außenumfang des Aufnahmebereiches 10a der Aufnahmevorrichtung 10 oder am Außenumfang der Infusionsleitung 4 ist eine Bildgebungsvorrichtung 11 mit einer Kamera angeordnet, die zur Erkennung 20 von Luftblasen oder anderen Gasblasen und vorzugsweise zur Erkennung von Partikeln in einer Infusionsflüssigkeit eingerichtet und ausgelegt ist. Die Bildgebungsvorrichtung 11 ist zur Erkennung einer Größe und zur Erkennung einer Veränderung der Größe (Vergrößerung/Verkleinerung) der Gasblasen eingerichtet und ausgelegt. Weiterhin ist die Bildgebungsvorrichtung 11 zur Erkennung der Bewegung der Gasblasen und vorzugsweise der Partikel eingerichtet und ausgelegt. Ergänzend ist ein Ultraschallsensor 13 vorgesehen, der vorzugsweise eine Initialisierung 18 durch Erkennung der Infusionsflüssigkeit in der Infusionsleitung 4 und gegebenenfalls die Gasblasenerkennung 20 übernimmt.

Die Kamera kann für sichtbares Licht ausgelegt sein oder auch für unsichtbares Licht, z.B. Infrarot.

### Bezugszeichenliste:

- 1: Infusionspumpe
- 2: Frontklappe
- 4: Infusionsleitung / (mehrteiliger) Infusionsschlauch
- 4a: Pumpensegment
- 4b: Sensorsegment
- 6: Beutel
- 8: Patient
- 10: Aufnahmevorrichtung
- 10a: Aufnahmebereich
- 10b: Backe
- 11: Bildgebungsvorrichtung
- 12: Kupplung
- 13: Ultraschallsensor
- 14: Klemme
- 16: elektronische Steuereinheit
- 18: Initialisierung
- 20: Gasblasenerkennung

## Patentansprüche

1. Infusionspumpe (1), die eine Infusionsleitung (4) oder eine Aufnahmevorrichtung (10) mit einem Aufnahmebereich (10a) für eine Infusionsleitung (4) aufweist, **dadurch gekennzeichnet, dass** am Außenumfang der Infusionsleitung (4) oder des Aufnahmebereiches (10a) ein Ultraschallsensor (13) und eine Bildgebungsvorrichtung (11) mit einer Kamera angeordnet sind, wobei zumindest die Bildgebungsvorrichtung (11) zur Erkennung von Luftblasen oder anderen Gasblasen und vorzugsweise zur Erkennung von Partikeln in einer Infusionsflüssigkeit eingerichtet und ausgelegt ist.

2. Infusionspumpe (1) nach Anspruch 1, **dadurch gekennzeichnet, dass** zumindest die Bildgebungsvorrichtung (11) zur Erkennung einer Größe und vorzugsweise zur Erkennung einer Veränderung der Größe der Gasblasen und/oder zur Erkennung der Bewegung der Gasblasen und vorzugsweise der Partikel eingerichtet und ausgelegt ist.

3. Infusionspumpe (1) nach Anspruch 1 oder 2, **dadurch gekennzeichnet, dass** der Ultraschallsensor (13) zur Initialisierung (18), umfassend eine Erkennung der Infusionsflüssigkeit in der Infusionsleitung (4), und/oder zur Erkennung von Luftblasen oder anderen Gasblasen - und vorzugsweise zur Erkennung von Partikeln - in der Infusionsflüssigkeit eingerichtet und ausgelegt ist.

4. Infusionspumpe (1) nach einem der vorhergehenden Ansprüche, **dadurch gekennzeichnet, dass** die Bildgebungsvorrichtung (11) eine Lichtquelle hat, die am Außenumfang des Aufnahmebereiches (10a) oder am Außenumfang der Infusionsleitung (4), vorzugsweise an einer der Kamera gegenüberliegenden Seite des Aufnahmebereiches (10a) oder der Infusionsleitung (4), angeordnet ist.

5. Infusionspumpe (1) nach einem der Ansprüche 1 bis 3, **dadurch gekennzeichnet, dass** die Kamera zur Erfassung von Infrarotstrahlung eingerichtet und ausgelegt ist.

6. Infusionspumpe (1) nach einem der vorhergehenden Ansprüche, **dadurch gekennzeichnet, dass** die Infusionsleitung (4) lichtdurchlässig oder lichtabsorbierend ist.

7. Infusionspumpe (1) nach einem der vorhergehenden Ansprüche mit einem Gehäuse, an dem eine Frontklappe (2) angelenkt ist, von der die Bildgebungsvorrichtung (11) abdeckbar ist.

8. Infusionspumpe (1) nach einem der vorhergehenden Ansprüche, **dadurch gekennzeichnet, dass** der Ultraschallsensor (13) für eine oder mehrere Frequenzen ausgelegt ist.

9. Infusionspumpe (1) nach einem der vorhergehenden Ansprüche, die als Peristaltikpumpe ausgestaltet ist, oder die eine Peristaltikpumpe aufweist, wobei die Infusionsleitung (4) der Infusionsschlauch (4) ist.

10. Infusionspumpe (1) nach einem der vorhergehenden Ansprüche **dadurch gekennzeichnet, dass** die Bildgebungsvorrichtung (11) zur Initialisierung (18), umfassend eine Erkennung der Infusionsflüssigkeit in der Infusionsleitung (4), eingerichtet und ausgelegt ist.

11. Verfahren zum Betrieb einer Infusionspumpe (1), vorzugsweise nach einem der vorhergehenden Ansprüche, **dadurch gekennzeichnet, dass** zunächst eine Initialisierung (18) dadurch erfolgt, dass ein Ultraschallsensor (13) oder eine Bildgebungsvorrichtung (11) eine Infusionsflüssigkeit in einer Infusionsleitung (4) erkennt, und dass danach eine Gasblasenerkennung (20) durch den Ultraschallsensor (13) und/oder durch die Bildgebungsvorrichtung (11) erfolgt.

12. Verfahren nach Anspruch 11, **dadurch gekennzeichnet, dass** eine Erkennung einer Größe und vorzugsweise einer Veränderung der Größe der Gasblasen und/oder eine Erkennung der Bewegung der Gasblasen und vorzugsweise der Partikel erfolgt/erfolgen.

13. Verfahren nach Anspruch 11 oder 12, **dadurch gekennzeichnet, dass** die Initialisierung (18) durch den Ultraschallsensor (13) und danach die Gasblasenerkennung (20) durch die Bildgebungsvorrichtung (11) erfolgt.

14. Verfahren nach Anspruch 13, **dadurch gekennzeichnet, dass** die Gasblasenerkennung (20) ergänzend auch durch den Ultraschallsensor (13) erfolgt.

15. Verfahren nach einem der Ansprüche 11 bis 14, **dadurch gekennzeichnet, dass** während der Gasblasenerkennung (20) auch eine Partikelerkennung erfolgt.

16. Computerimplementiertes Speichermedium oder elektronische Steuereinheit (16), das/die dazu ausgelegt und eingerichtet und programmiert ist, das Verfahren nach einem der Ansprüche 11 bis 15 auszuführen.
